# EUROPEAN PATENT SPECIFICATION

(11) **EP 3 859 335 B1**
(45) Date of publication and mention of the grant of the patent: **02.10.2024**
(21) Application number: 19865578.9
(22) Date of filing: 20.08.2019
(51) Int. Cl.: G01N 33/543, G01N 33/547, G01N 37/00, G01N 33/558

(54) **MEMBRANE CARRIER FOR TEST KIT AND TEST KIT**
MEMBRANTRÄGER FÜR TESTKIT UND TESTKIT
SUPPORT DE MEMBRANE POUR KIT D'ESSAI ET KIT D'ESSAI

(30) Priority: 25.09.2018 JP 2018179125
(43) Date of publication of application: 04.08.2021
(73) Proprietor: Denka Company Limited, Tokyo 103-8338 (JP)
(72) Inventor: AOYAMA, Shuhei, Machida-city, Tokyo 194-8560 (JP); AKIYAMA, Yuto, Machida-city, Tokyo 194-8560 (JP)
(74) Representative: Schnappauf, Georg
(86) International application number: PCT/JP2019/032447
(87) International publication number: WO 2020/066369

(56) References cited:
- WO-A1-2016/098740
- WO-A1-2016/098740
- WO-A1-2017/217406
- WO-A1-2018/181540
- JP-A- 2004 101 516
- JP-A- 2004 101 516
- JP-A- 2007 147 602
- JP-A- 2007 147 602
- JP-A- 2008 020 412
- JP-A- 2008 020 412
- JP-A- 2014 066 532
- JP-A- 2017 044 482
- JP-A- 2017 044 482
- US-A1- 2009 017 518
- AOYAMA SHUHEI ET AL: "Thermally imprinted microcone structure-assisted lateral-flow immunoassay platforms for detecting disease marker proteins", ANALYST, vol. 144, no. 5, 7 March 2019 (2019-03-07), UK, pages 1519 - 1526, XP055853781, ISSN: 0003-2654, DOI: 10.1039/C8AN01903G
- AOYAMA SHUHEI ET AL: "Enhanced Immunoadsorption on Imprinted Polymeric Microstructures with Nanoengineered Surface Topography for Lateral Flow Immunoassay Systems | Analytical Chemistry", ANAL. CHEM., vol. 91, 4 October 2019 (2019-10-04), pages 13377 - 13382, XP055853773

## Description

### TECHNICAL FIELD

The present invention relates to a membrane carrier for a test kit and a test kit.

### BACKGROUND ART

In recent years, a Point of Care Testing (POCT) reagent, which measures an affliction of an infectious disease, pregnancy, a blood glucose level, and the like by using an antigen-antibody reaction and the like, has attracted attention. The POCT reagent is, for example, a reagent for a test performed by a subject or a test performed by the subj ect himself/herself, and has characteristics that a result can be determined in a short time, a use method is simple, and cost is low. With these characteristics, the POCT reagent is frequently used for medical examinations and periodic medical examinations at a stage where the symptoms are mild, and is an important medical examination tool in home medical care, which is expected to increase in the future.

In many POCT reagents, a determination is made by introducing a liquid sample such as blood into a test kit and detecting a specific substance to be detected contained therein. As a method for detecting a specific substance to be detected from the liquid sample, an immunochromatography method is often used. An immunochromatography method refers to a technique in which a substance to be detected and a labeling substance are bound while a liquid dropped on a membrane carrier for a test kit moves on the membrane carrier, these specifically bind to a substance (hereinafter, referred to as detection substance) immobilized in the test kit, and a change in color or mass generated as a result is detected. The detection substance may be referred to as a reagent.

A nitrocellulose membrane is often used as a membrane carrier for moving a liquid sample The nitrocellulose membrane has a large number of fine holes having a diameter of about several um, and the liquid sample moves in the holes by capillary force.

However, since the nitrocellulose membrane is derived from a natural product and a hole diameter and a way of connecting the holes are not uniform, there occurs a difference in a flow rate of the liquid sample flowing in each membrane. In a case where a difference occurs in the flow rates, the time required to detect a substance to be detected is also changed, and as a result, the substance to be detected may be erroneously determined as non-detected before binding occurs.

In order to solve the above-mentioned problem, a liquid sample test kit of artificially preparing a fine flow channel has been prepared (Patent Document 1). In Patent Document 1, by using a synthetic material, it is possible to prepare a membrane carrier having a uniform structure. Therefore, it is possible to decrease a possibility of erroneously determining a substance to be detected as non-detection before binding occurs.

In a case where a synthetic material is used, it is necessary to increase the affinity between the detection substance and the material in order to improve the detection sensitivity, and it is considered effective to perform various surface treatments on the material in advance (Patent Document 2). Patent Document 3 discloses a membrane carrier for a liquid sample test kit for detecting a substance to be detected in a liquid sample, in which at least one flow channel capable of transporting the liquid sample is provided, and the microstructure that generates a capillary action for transporting the liquid sample is provided on a bottom surface of the flow channel. Patent Document 4 discloses a membrane carrier comprising a channel whith microstructures forming a rough structure having protrusions.

### RELATED DOCUMENT

### PATENT DOCUMENT

[Patent Document 1]
   Japanese Patent No. 5799395
[Patent Document 2]
   US Patent Application Publication No. 2011/0284110
[Patent Document 3
   ] International Publication No. WO 2016/098740
[Patent Document 4] International Publication No. WO 2017/217406

### SUMMARY OF THE INVENTION

### TECHNICAL PROBLEM

Further improvement in detection sensitivity is required for the above-mentioned test kits.

The present invention has been made in view of the above circumstances and is to provide a membrane carrier for a test kit capable of obtaining a test kit with improved detection sensitivity and a test kit with improved detection sensitivity.

### SOLUTION TO PROBLEM

The present inventors have made extensive studies to solve the above technical problem. As a result, it was found that by setting a maximum peak height Rp of a roughness curve and an average length RSm of roughness curve elements to be within a specific range, and providing a rough structure having protrusions to which a silane coupling agent is attached in a flow channel of the membrane carrier, the detection sensitivity of a substance to be detected in a liquid sample can be improved, thereby achieving the present invention.

That is, according to the present invention, a membrane carrier for a test kit and a test kit are provided as follows.

[1] A membrane carrier for a test kit for detecting a substance to be detected in a liquid sample, the membrane carrier including:
   a flow channel having a detection zone,
   in which the flow channel has a rough structure A having protrusions,
   at least in the detection zone, a rough microstructure B is formed on a surface of the protrusion,
   a silane coupling agent is attached to a surface of the rough microstructure B, and
   a maximum peak height Rp of a roughness curve of a protrusion at which the rough microstructure B is formed, as measured according to JIS B0601: 2013 using a three-dimensional roughness analysis scanning electron microscope, is equal to or more than 0.005 um and equal to or less than 10 µm, and an average length RSm of a roughness curve element is equal to or more than 0.01 µm and equal to or less than 15 µm.
[2] The membrane carrier for a test kit according to [1],
   in which the silane coupling agent includes at least one selected from the group consisting of a silane coupling agent having an amino group, a silane coupling agent having a mercapto group, a silane coupling agent having an epoxy group, a silane coupling agent having an acrylic group, and a silane coupling agent having a methacryl group, a silane coupling agent having a vinyl group, and a silane coupling agent having an isocyanate group.
[3] The membrane carrier for a test kit according to [1] or [2],
   in which a cross-linking agent is further attached to the surface of the rough microstructure B.
[4] The membrane carrier for a test kit according to [3],
   in which the cross-linking agent includes at least one selected from glutaraldehyde, dextran, 1,4-phenyldiisocyanate, toluene-2,4 diisocyanate, polyethyleneimine, 1-ethyl-3-(3-dimethylaminopropyl)carbodiimide, N-succinimidyl 4-(N-maleimide methyl)cyclohexane-1-carboxylate, and N-hydroxysucciimide.
[5] The membrane carrier for a test kit according to any one of [1] to [4],
   in which the rough structure A includes a thermoplastic resin.
[6] The membrane carrier for a test kit according to [5],
   in which the thermoplastic resin includes at least one selected from a polyester-based resin, a polyolefin-based resin, a polystyrene-based resin, a polycarbonate-based resin, a fluorine-based resin, and a (meth)acrylic-based resin.
[7] The membrane carrier for a test kit according to any one of [1] to [6],
   in which an average height of the protrusions is equal to or more than 5 um and equal to or less than 1,000 µm.
[8] The membrane carrier for a test kit according to any one of [1] to [7],
   in which an average distance between the adjacent protrusions in the rough structure A is equal to or less than 500 µm.
[9] The membrane carrier for a test kit according to any one of [1] to [8],
   in which an average diameter of a bottom surface of the protrusion is equal to or more than 5 µm and equal to or less than 1,000 µm.
[10] A test kit having the membrane carrier according to any one of [1] to [9].

### ADVANTAGEOUS EFFECTS OF INVENTION

According to the present invention, it is possible to provide a membrane carrier for a test kit capable of obtaining a test kit with improved detection sensitivity and a test kit with improved detection sensitivity.

### BRIEF DESCRIPTION OF THE DRAWINGS

Fig. 1 is an example of an embodiment according to the present invention, and is a schematic top view of a test kit.
Fig. 2 is an example of an embodiment according to the present invention, and is a schematic top view of a membrane carrier.
Fig. 3(a) is an example of an embodiment according to the present invention, and is a plane view (top view) of a rough structure A, and Fig. 3(b) is a perspective view of a protrusion constituting the rough structure A shown in Fig. 3(a).
Fig. 4 (a) is an example of an embodiment according to the present invention, and is a plane view (top view) of the rough structure A, and Fig. 4(b) is a perspective view of a protrusion constituting the rough structure A shown in Fig. 4(a).
Fig. 5(a) is an example of an embodiment according to the present invention, and is a plane view (top view) of the rough structure A, and Fig. 5(b) is a perspective view of a protrusion constituting the rough structure A shown in Fig. 5(a).
Fig. 6 (a) is an example of an embodiment according to the present invention, and is a plane view (top view) of the rough structure A, and Fig. 6(b) is a perspective view of a protrusion constituting the rough structure A shown in Fig. 6(a).
Fig. 7 is an example of an embodiment according to the present invention, and is a schematic top view of the rough structure A.
Fig. 8 is an example of an embodiment according to the present invention, and is a schematic view of surface treatment.
Fig. 9 is an example of an embodiment according to the present invention, and is a diagram showing an electron micrograph showing an enlarged view of a protrusion at which a rough microstructure B is formed.

### DESCRIPTION OF EMBODIMENTS

Hereinafter, embodiments of the present invention will be described with reference to the drawings. In addition, the drawings are schematic views, and do not match the actual dimensional ratio.

### 1. Membrane carrier

The membrane carrier for a test kit according to the present embodiment (also simply referred to as "membrane carrier 3") is a membrane carrier for a test kit for detecting a substance to be detected in a liquid sample, can transport the liquid sample, and has a flow channel having a detection zone, in which the flow channel can generate a capillary action for transporting the liquid sample, and has a rough structure A having a protrusion.

At least in the detection zone of the membrane carrier for a test kit according to the present embodiment, a rough microstructure B is formed on a surface of the protrusion, a silane coupling agent is attached to a surface of the rough microstructure B, a maximum peak height Rp of a roughness curve of the protrusion at which the rough microstructure B is formed, as measured according to JIS B0601: 2013 using a three-dimensional roughness analysis scanning electron microscope, is equal to or more than 0.005 um and equal to or less than 10 µm, and an average length RSm of a roughness curve element is equal to or more than 0.01 µm and equal to or less than 15 µm.

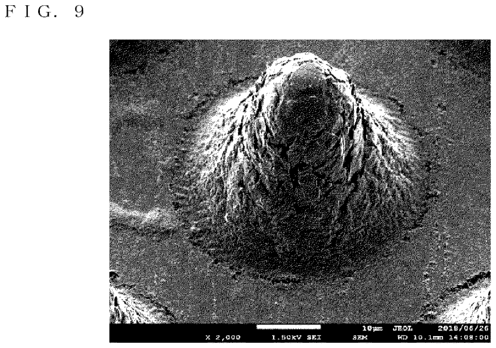

In addition, the rough microstructure B formed on the surface of the protrusion according to the present embodiment has a macroscopic shape such as a striped shape or a streak shape, as shown in Fig. 9, for example.

At least in the detection zone of the membrane carrier for a test kit according to the present embodiment, as Rp and RSm are within the above ranges and a rough microstructure B to which a silane coupling agent is attached is formed, it is possible to increase an amount of a substance to be detected carried in the detection zone, and as a result, it is possible to improve the detection sensitivity of the substance to be detected in the liquid sample.

The reason why the amount of the detection substance carried in the detection zone can be increased is not clear, but the following reasons are considered.

First, the maximum peak height Rp of the roughness curve is a value indicating the maximum height of a convex portion in the rough microstructure B, and the average length RSm of the roughness curve element indicates an average value of a period of roughness (concavity and convexity) in the rough microstructure B.

Therefore, the Rp and RSm are indices of balance between an interval of the formed roughness and a height of the roughness in the rough microstructure B.

Therefore, at least in the detection zone, the rough microstructure B in which Rp and RSm are within the above ranges is considered to have a structure having a space suitable for carrying the detection substance. Therefore, since the membrane carrier for a test kit according to the present embodiment has a rough microstructure B in which Rp and RSm are within the above ranges at least in the detection zone, it is considered that it is possible to increase the amount of the detection substance carried in the detection zone.

In addition, it is considered that by attaching the silane coupling agent to the rough microstructure B, it is possible to carry a detection substance that cannot be adsorbed well on the surface of the rough microstructure B due to steric hindrance or the like.

From the above, it is considered that the membrane carrier for a test kit according to the present embodiment increases the amount of the detection substance carried in the detection zone by a synergistic effect of the rough microstructure B in which Rp and RSm are within the ranges and the silane coupling agent.

In the membrane carrier for a test kit according to the present embodiment, a silane coupling agent is attached to the surface of the rough microstructure B. Here, the silane coupling agent may be physically adsorbed on the surface of the rough microstructure B, may be chemically adsorbed, or may be directly bonded on a functional group existing on the surface of the rough microstructure B.

Examples of the silane coupling agent according to the present embodiment include a silane coupling agent having an amino group, a silane coupling agent having a mercapto group, a silane coupling agent having an epoxy group, a silane coupling agent having an acrylic group, a silane coupling agent having a methacryl group, a silane coupling agent having a vinyl group, and a silane coupling agent having an isocyanate group.

Examples of the silane coupling agent having an amino group include 3-aminopropyltriethoxysilane, 3-aminopropyltrimethoxysilane, 3-(2-aminoethyl) aminopropyltriethoxysilane, 3-(2-aminoethyl) aminopropyltrimethoxysilane, and the like.

Examples of the silane coupling agent having a mercapto group include 3-mercaptopropyltrimethoxysilane, 3-mercaptopropyltriethoxysilane, 2-mercaptoethyltrimethoxysilane, 2-mercaptoethyltriethoxysilane, and the like.

Examples of the silane coupling agent having an epoxy group include 3-glycidoxypropyltrimethoxysilane, 5,6-epoxyhexyltriethoxysilane, 2-(3,4-epoxycyclohexyl) ethyltrimethoxysilane, 3-glycidoxypropyltriethoxysilane, and the like.

Examples of the silane coupling agent having an acrylic group include 3-acryloxypropyltrimethoxysilane, 3-acryloxypropylmethyldimethoxysilane, 3-acryloxypropylmethyldiethoxysilane, 3-acryloxypropyltriethoxysilane, and the like.

Examples of the silane coupling agent having a methacryl group include 3-methacryloxypropylmethyldimethoxysilane, 3-methacryloxypropyltrimethoxysilane, 3-methacryloxypropylmethyldiethoxysilane, 3-methacryloxypropyltriethoxysilane, γ-(methacryloyloxypropyl) trimethoxysilane, γ-(methacryloyloxypropylmethyl) dimethoxysilane, and the like.

Examples of the silane coupling agent having a vinyl group include vinyltriethoxysilane, vinyltrimethoxysilane, and the like.

Examples of the silane coupling agent having an isocyanate group include trimethoxysilylmethyl isocyanate, triethoxysilylmethyl isocyanate, tripropoxysilylmethyl isocyanate, 2-trimethoxysilylethyl isocyanate, 2-triethoxysilylethyl isocyanate, 2-tripropoxysilylethyl isocyanate, 3-trimethoxysilylpropyl isocyanate, 3-triethoxysilylpropyl isocyanate, 3-tripropoxysilylpropyl isocyanate, 4-trimethoxysilylbutylisocyanate, 4-triethoxysilylbutylisocyanate, 4-tripropoxysilylbutylisocyanate, and the like.

These silane coupling agents may be used alone or in combination of two or more.

Among these, at least one selected from a silane coupling agent having an amino group and a silane coupling agent having an epoxy group is preferable from a viewpoint of capable of further increasing the amount of the detection substance carried in the detection zone.

The amount of the silane coupling agent attached is, for example, in a range of equal to or more than 0.1 mg and equal to or less than 10 g per 1 m² of the surface area of a membrane carrier 3.

In the membrane carrier for a test kit according to the present embodiment, it is preferable that a cross-linking agent (crosslinker) is further attached to the surface of the rough microstructure B. By further attaching the cross-linking agent to the surface of the rough microstructure B, the detection substance can be stably carried on the surface of the rough microstructure B. With this, it is possible to further increase the amount of the detection substance carried in the detection zone.

In addition, by using the cross-linking agent, it becomes possible to carry a detection substance that cannot be well adsorbed on the surface of the rough microstructure B due to steric hindrance or the like, and thus it is possible to further increase the amount of the detection substance carried.

Here, the cross-linking agent may be physically adsorbed on the surface of the rough microstructure B, may be chemically adsorbed, or may be directly bonded to the functional group existing on the surface of the rough microstructure B. In addition, the cross-linking agent may be physically adsorbed on the surface of a layer containing the silane coupling agent, may be chemically adsorbed, or may be directly bonded to the silane coupling agent.

Examples of the cross-linking agent according to the present embodiment include formaldehyde, glutaraldehyde, dextran, 1,4-phenyldiisocyanate, toluene-2,4 diisocyanate, polyethyleneimine, hexamethylene diisocyanate, hexamethylene diisothiocyanate, N, N'-polymethylene bisiodoacetamide, N, N'-ethylene bismaleimide, ethylene glycol bissuccinimidyl succinate, bisdiazobenzidine, 1-ethyl-3-(3-dimethylaminopropyl) carbodiimide, succinimidyl 3-(2-pyridyldithio) propionate, N-succinimidyl 4-(N-maleimide methyl) cyclohexane-1-carboxylate, N-hydroxysucciimide, N-sulfosuccinimidyl 4-(N-maleimide methyl) cyclohexane-1-carboxylate, N-succinimidyl (4-iodoacetyl) aminobenzoate, N-succinimidyl 4-(1-maleimidephenyl) butyrate, N-(ε-maleimide caproyloxy) succinimide, iminothiolane, S-acetylmercaptosuccinic anhydride, methyl-3-(4'-dithiopyridyl) propion imidate, methyl-4-mercaptobutyryl imidate, methyl-3-mercaptopropion imidate, N-succinimidyl-S-acetyl mercaptoacetate, and the like.

These cross-linking agents may be used alone or in combination of two or more.

Among these, at least one cross-linking agent selected from glutaraldehyde, dextran, 1,4-phenyldiisocyanate, toluene-2,4 diisocyanate, polyethyleneimine, 1-ethyl-3-(3-dimethylaminopropyl)carbodiimide, N-succinimidyl 4-(N-maleimide methyl)cyclohexane-1-carboxylate, and N-hydroxysucciimide is preferable from a viewpoint of capable of further increasing the amount of the detection substance carried in the detection zone.

The amount of the cross-linking agent attached is, for example, in a range of equal to or more than 0.1 mg and equal to or less than 10 g per 1 m² of the surface area of the membrane carrier 3.

Here, the substance to be detected is not limited at all, and may be any substance that can have an antigen-antibody reaction with an antibody, such as various pathogens and various clinical markers. Specific examples of the substance to be detected include virus antigens such as influenza virus, norovirus, adenovirus, RS virus, HAV, HBs, and HIV, bacterial antigens such as MRSA, group A streptococcus, group B streptococcus, and genus Legionella bacteria, toxins produced by bacteria, mycoplasma, chlamydia trachomatis, hormones such as human chorionic gonadotropin, C-reactive protein, myoglobin, cardiac troponin, various tumor markers, agrochemicals, and environmental hormones, but are not limited thereto. More specifically, the substance to be detected is more useful for a case of requiring urgent detection and treatment such as influenza virus, norovirus, C-reactive protein, myoglobin, and cardiac troponin. The substance to be detected may be an antigen capable of inducing an immune reaction alone, or a hapten not capable of inducing an immune reaction by itself but capable of binding to an antibody by an antigen-antibody reaction. The substance to be detected is usually in a state of being floated or dissolved in the liquid sample. The liquid sample may be a sample in which the substance to be detected is floated or dissolved in a buffer solution, for example.

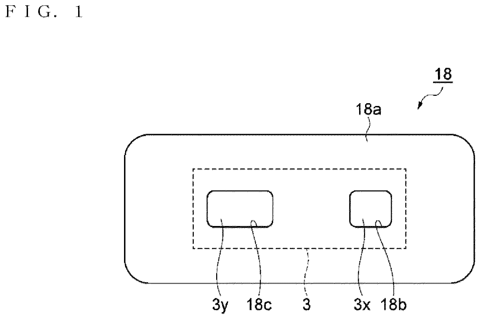

A liquid sample test kit according to the present embodiment (hereinafter, also simply referred to as "test kit 18") has the membrane carrier for a test kit according to the present embodiment and detects a substance to be detected in the liquid sample. Fig. 1 is a schematic top view of the test kit. For example, as shown in Fig. 1, the test kit 18 includes a membrane carrier 3 and a housing 18a that houses the membrane carrier 3. The membrane carrier 3 has, on a surface thereof, a dropping zone 3x in which a liquid sample is dropped, and a detection zone 3y for detecting the substance to be detected in the liquid sample. The dropping zone 3x is exposed at a first opening 18b of the housing 18a. The detection zone 3y is exposed at a second opening 18c of the housing 18a.

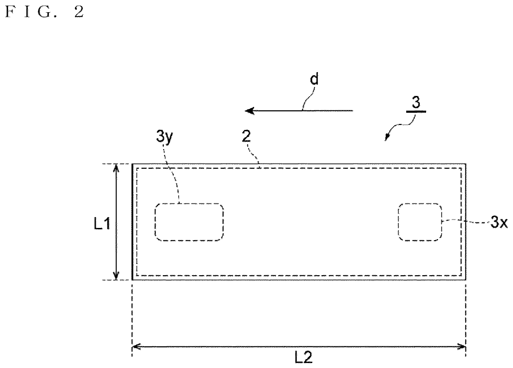

Fig. 2 is a schematic top view of the membrane carrier 3. As shown in Fig. 2, the membrane carrier 3 includes at least one flow channel 2 that transports a liquid sample. A rough structure A is provided on a bottom surface of the flow channel 2 (not shown, details will be described later). The rough structure A is positioned at least between the dropping zone 3x and the detection zone 3y. The rough structure A may be provided over the entire surface of the membrane carrier 3. The entire surface of the membrane carrier 3 may be the flow channel 2 of the liquid sample. The rough structure A causes capillary action. Due to the capillary action of the rough structure A, the liquid sample is transported to the detection zone 3y (along a transporting direction d) from the dropping zone 3x via the rough structure A. In a case where the substance to be detected in the liquid sample is detected in the detection zone 3y, color of the detection zone 3y changes.

The entire shape of the membrane carrier 3 is not limited, but may be, for example, a polygon such as a square, a circle, or an ellipse. In a case where the membrane carrier 3 is a square, a vertical width (length in a short-side direction) L1 of the membrane carrier 3 may be equal to or more than 2 mm and equal to or less than 100 mm, for example, and a horizontal width (length in a long-side direction) L2 of the membrane carrier 3 may be equal to or more than 2 mm and equal to or less than 100 mm, for example. A thickness of the membrane carrier excluding a height of the rough structure A may be equal to or more than 0.1 mm and equal to or less than 10 mm, for example.

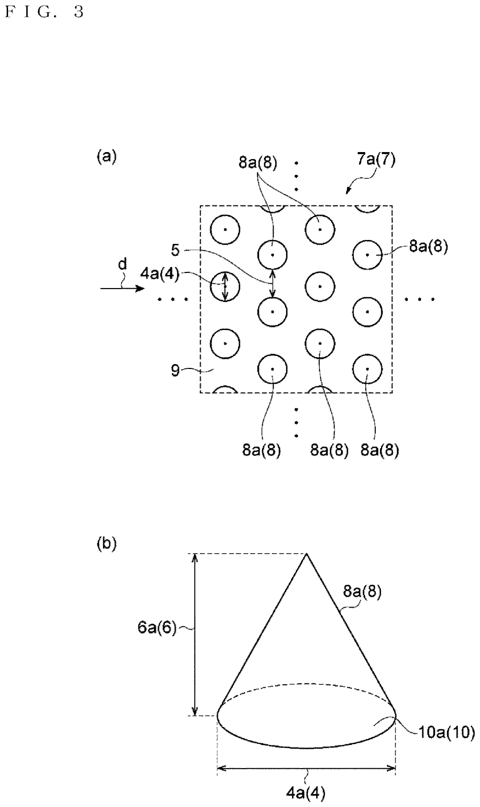

Figs. 3 to 6 each show an example of a rough structure A provided on a bottom surface of the flow channel and a protrusion (also referred to as convex portion) constituting thereof in the present embodiment. In Figs. 3 to 6, Figs. (a) each are a plane view (top view) of the rough structure A, and Figs. (b) each are a perspective view of the protrusion constituting the rough structure A shown in Figs. (a). As shown in Figs. 3 to 6, a rough structure A (7) is a collective of protrusions 8. That is, the membrane carrier 3 includes a flat portion 9 corresponding to the bottom surface of the flow channel 2 for the liquid sample, and a plurality of protrusions 8 protruding from the flat portion 9. By the capillary action, a space between the plurality of the protrusions 8 functions as the flow channel 2 transporting the liquid sample along the surface of the membrane carrier 3. In other words, a void in the rough structure A (7) functions as the flow channel 2 transporting the liquid sample along the surface of the membrane carrier 3 by the capillary action. The plurality of the protrusions 8 may be regularly or translationally symmetrically arranged in line on the surface of the membrane carrier 3.

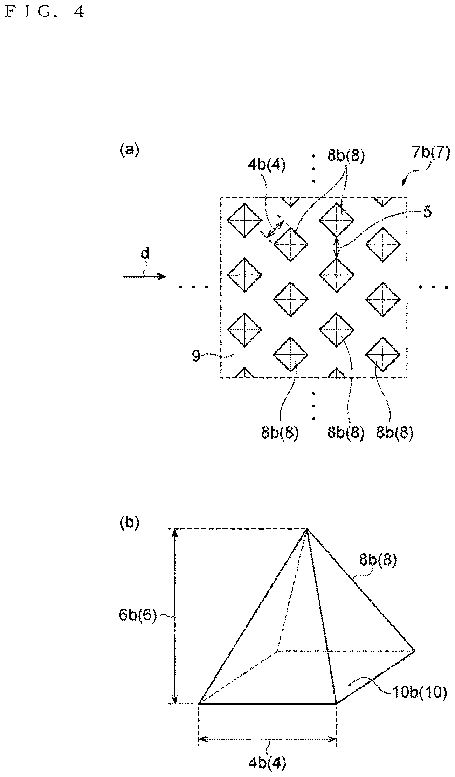

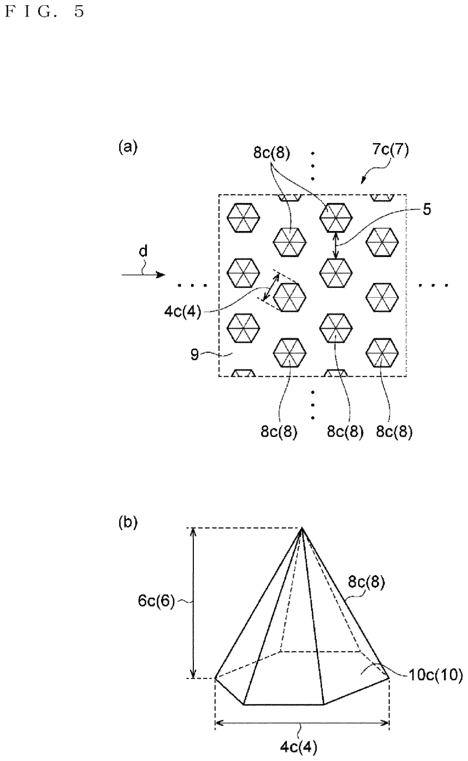

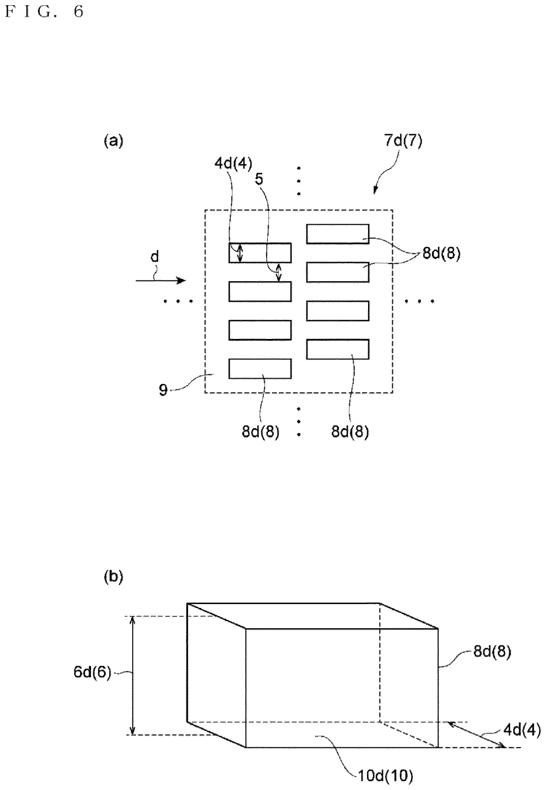

The shape of the plurality of the protrusions 8 constituting the rough structure A (7) can be freely selected. Examples of the shape of the protrusion 8 include a cone, a polygonal pyramid, a truncated cone, a truncated polypyramid, a cylinder, a polygonal prism, a hemisphere, and a hemi-ellipsoid. Examples of the bottom surface of the rough structure A include a circle or a polygon (for example, a square, a rhombus, a rectangle, a triangle, a hexagon, and the like). For example, as shown in Fig. 3, the shape of the protrusion 8a may be a cone. For example, as shown in Fig. 4, the shape of the protrusion 8b may be a quadrangular pyramid. For example, as shown in Fig. 5, the shape of the protrusion 8c may be a hexagonal pyramid. For example, as shown in Fig. 6, the shape of the protrusion 8d may be a quadrangular prism (a line and space structure in which the protrusion 8d is in a line shape). From a viewpoint that the entire surface of the membrane carrier 3 can be visually recognized at a time when the rough structure A (7) is viewed from a plane view (viewed from above), and a change in color at a time when the substance to be detected has been detected is easily confirmed by an optical technique, among these, a conical body structure such as a cone or a polygonal pyramid is suitable as the shape of the protrusion 8. Among the conical body structures, cones are preferable.

The shape of the protrusion 8 constituting the rough structure A (7) does not need to be a geometrically accurate shape, and may be a shape with rounded corners or a shape with fine roughness on the surface.

A diameter 4 (average diameter) of the bottom surface 10 of the protrusion 8 constituting the rough structure A (7) is preferably equal to or more than 5 µm and equal to or less than 1000 µm, and more preferably equal to or more than 10 µm and equal to or less than 500 µm. In a case where the diameter 4 of the bottom surface 10 of the protrusion 8 is equal to or more than the lower limit value, the accuracy of fine processing can be suppressed low, and a cost for forming the rough structure A (7) tends to be low. In a case where the diameter 4 of the bottom surface 10 of the protrusion 8 is equal to or less than the upper limit value, the number of protrusions 8 in one test kit increases, and the liquid sample can be easily developed.

Here, for the diameter 4 of the bottom surface 10 of the protrusion 8 is, for example, it is possible to select five optional protrusions 8 from the rough structure A (7), and to employ an average value of the diameter of the bottom surface 10 of the selected five protrusions 8.

The diameter 4 of the bottom surface 10 of the protrusion 8 is defined as a representative length of the bottom surface 10 of the protrusion 8. The representative length of the bottom surface 10 is a diameter in a case where the shape of the bottom surface 10 is a circle, a shortest side length in a case where the shape of the bottom surface 10 is a triangle or a quadrangle, a longest diagonal length in a case where the shape of the bottom surface 10 is a polygon that is more than a pentagon, and a longest length of the bottom surface 10 in a case where the shape of the bottom surface 10 is another shape.

As shown in Fig. 3, in a case where the shape of the protrusion 8a is a cone, the diameter 4a of the bottom surface 10a of the protrusion 8a is a diameter of the bottom surface (circle) of the cone. As shown in Fig. 4, in a case where the shape of the protrusion 8b is a regular quadrangular pyramid, the diameter 4b of the bottom surface 10b of the protrusion 8b is a length of a side of the bottom surface (regular square) 10b. As shown in Fig., in a case where the shape of the protrusion 8c is a regular hexagonal pyramid, the diameter 4c of the bottom surface 10c of the protrusion 8c is the length of a diagonal line passing through the center of the bottom surface (regular hexagon) 10c (the length of the longest diagonal line). As shown in Fig. 6, in a case where the shape of the protrusion 8d is a rectangle, the diameter 4d of the bottom surface 10d of the protrusion 8d is a length of the shortest side of the bottom surface (rectangle) 10d (in Fig. 6, the length in a direction orthogonal to the transporting direction of the liquid sample).

A height 6 (average height) of the protrusion 8 constituting the rough structure A (7) is preferably equal to or more than 5 µm and equal to or less than 1000 µm, and more preferably equal to or more than 10 µm and equal to or less than 500 µm. In a case where the height 6 of the protrusion 8 is equal to or more than the lower limit value, a volume of the flow channel 2 increases, and the liquid sample can be developed in a shorter time. In a case where the height 6 of the protrusion 8 is equal to or less than the upper limit value, a time and cost for preparing the rough structure A (7) can be reduced, and the rough structure A (7) can be more easily prepared.

Here, for the height 6 of the protrusion 8, for example, it is possible to select five optional protrusions 8 from the rough structure A (7), and to employ an average value of the height of the five selected protrusions 8.

The height 6 of the protrusion 8 is defined as a maximum length of the protrusion 8 in a direction orthogonal to the flat portion 9. As shown in Fig. 3, in a case where the shape of the protrusion 8a is a cone, the height 6a of the protrusion 8a is the maximum length of the protrusion 8a in the direction orthogonal to the flat portion 9 (the height of the cone) . As shown in Fig. 4, in a case where the shape of the protrusion 8b is a quadrangular pyramid, the height 6b of the protrusion 8b is the maximum length of the protrusion 8b in the direction orthogonal to the flat portion 9 (height of the quadrangular pyramid) . As shown in Fig. 5, in a case where the shape of the protrusion 8c is a hexagonal pyramid, the height 6c of the protrusion 8c is the maximum length of the protrusion 8c in the direction orthogonal to the flat portion 9 (height of the hexagonal pyramid). As shown in Fig. 6, in a case where the shape of the protrusion 8d is a quadrangular prism, the height 6d of the protrusion 8d is the maximum length of the protrusion 8d in the direction orthogonal to the flat portion 9 (the height of the quadrangular prism).

A distance 5 (average distance) between the adjacent protrusions in the rough structure A (7), that is, the closest distance between the protrusions 8 is preferably equal to or more than 0 and equal to or less than 500 µm. It is preferably equal to or less than 500 µm, and more preferably equal to or more than 2 µm and equal to or less than 100 µm. The distance 5 between the adjacent protrusions cannot be less than 0 µm, and in a case where the distance 5 between the adjacent protrusions is equal to or less than the upper limit value, a contact area between the liquid sample and the flow channel 2 increases, and with this, the capillary force increases. Therefore, it becomes easier to move the liquid sample. Here, the "distance between adjacent protrusions" is the closest distance between a pair of adjacent protrusions 8.

Here, for the distance 5 between the adjacent protrusions is, for example, it is possible to select five distances between the five optional adjacent protrusions from the rough structure A (7), and to employ an average value of the distances between the five selected adjacent protrusions.

An aspect ratio of the protrusions 8 constituting the rough structure A (7) is preferably equal to or more than 0.1 and equal to or less than 10, and more preferably equal to or more than 0.1 and equal to or less than 2.0. The aspect ratio mentioned herein is a value (Lh/Lv) obtained by dividing the height 6 (Lh) of the protrusion 8 by the representative length (diameter 4) (Lv) of the bottom surface 10 of the protrusion 8. In a case where the aspect ratio is equal to or more than the lower limit value, the contact area between the liquid sample and the flow channel 2 increases, and with this, the capillary force increases. Therefore, it becomes easier to move the liquid sample. In a case where the aspect ratio is equal to or less than the upper limit value, the rough structure A can be more easily prepared.

The rough structure A (7) and the membrane carrier 3 of the test kit 18 according to the present embodiment include a thermoplastic resin, for example. In other words, by processing a membrane-shaped base material formed of a thermoplastic resin, it is possible to prepare a membrane carrier 3 having a rough structure A (7).

Examples of a processing method include thermal imprinting, UV imprinting, injection molding, etching, photolithography, mechanical cutting, laser processing, and the like. Among these, as a technique of performing accurate processing at a low cost, thermal imprinting on a thermoplastic resin is suitable.

Examples of the thermoplastic resin include a polyester-based resin, a polyolefin-based resin, a polystyrene-based resin, a polycarbonate-based resin, a fluorine-based resin, a (meth)acrylic-based resin, and the like, and specifically, various kinds such polyethylene terephthalate (PET), cycloolefin polymer (COP), polypropylene (PP), polystyrene (PS), polycarbonate (PC), polyvinylidene fluoride (PVDF), polymethyl methacrylate (PMMA), polyethylene (PE), and the like can be used. These thermoplastic resins may be used alone, or may be used in combination of two or more.

In a case of a processing method using a mold such as imprinting or injection molding, the conical body is thinner at the top than at the bottom. Therefore, a volume to be carved out during mold preparation is smaller than that of a column preparation with the same bottom surface, and thus it is possible to prepare a mold at a low cost. In this case, it is possible to perform detection of the substance to be detected in the liquid sample at a lower cost.

As described above, the membrane carrier 3 includes a rough structure A (7) provided on one surface of the membrane carrier 3, a flow channel 2 formed by the rough structure A (7) and for transporting a liquid sample, and a detection zone (detection portion) 3y for detecting the substance to be detected in the liquid sample. The membrane carrier 3 may be the membrane carrier 3 for the test kit 18 for detecting the substance to be detected in the liquid sample.

A maximum peak height Rp of the roughness curve of the protrusion at which the rough microstructure B is formed, as measured according to JIS B0601: 2013 using a three-dimensional roughness analysis scanning electron microscope, is equal to or more than 0.005 µm and equal to or less than 10 µm. The maximum peak height Rp is preferably equal to or more than 0.010 µm, more preferably equal to or more than 0.050 um, further more preferably equal to or more than 0.10 µm, and even more preferably equal to or more than 0.15 µm. The maximum peak height Rp is preferably equal to or less than 8.0 µm, more preferably equal to or less than 5.0 µm, further more preferably equal to or less than 3.0 µm, and even more preferably equal to or less than 1.0 µm.

An average value of the maximum peak height Rp of the roughness curve of the protrusion at which the rough microstructure B is formed, as measured according to JIS B0601: 2013 using a three-dimensional roughness analysis scanning electron microscope, is preferably equal to or more than 0.005 um and equal to or less than 10 µm. The average value of the maximum peak height Rp is preferably equal to or more than 0.010 µm, more preferably equal to or more than 0.050 µm, further more preferably equal to or more than 0.10 µm, and even more preferably equal to or more than 0.15 µm. The average value of the maximum peak height Rp is preferably equal to or less than 8.0 um, more preferably equal to or less than 5.0 µm, further more preferably equal to or less than 3.0 µm, and even more preferably equal to or less than 1.0 um.

For the average value of the maximum peak height Rp, it is possible to optionally select three protrusions 8 at which the rough microstructure B is formed, and to employ an average value of the maximum peak heights Rp of the three selected protrusions 8.

In the membrane carrier 3, the maximum peak height Rp of the roughness curve of the flat portion 9 may be equal to or more than 0.005 µm and equal to or less than 10 µm. The maximum peak height Rp of the roughness curve of the flat portion 9 is preferably equal to or more than 0.010 µm, more preferably equal to or more than 0.050 µm, further more preferably equal to or more than 0.10 µm, and even more preferably equal to or more than 0.15 µm. The maximum peak height Rp of the roughness curve of the flat portion 9 is preferably equal to or less than 8.0 µm, more preferably equal to or less than 5.0 µm, further more preferably equal to or less than 3.0 µm, and even more preferably equal to or less than 1.0 µm.

An average length RSm of the roughness curve element of the protrusion at which the rough microstructure B is formed, as measured according to JIS B0601: 2013 using a three-dimensional roughness analysis scanning electron microscope, is equal to or more than 0.01 µm and equal to or less than 15 µm. The average length RSm is preferably equal to or more than 0.03 µm, more preferably equal to or more than 0.05 µm, further more preferably equal to or more than 0.08 µm, and even more preferably equal to or more than 0.10 µm. The average length RSm is preferably equal to or less than 10 µm, more preferably equal to or less than 5.0 µm, and even more preferably equal to or less than 3.0 µm.

The average value of the average length RSm of the roughness curve element of the protrusion at which the rough microstructure B is formed, as measured according to JIS B0601: 2013 using a three-dimensional roughness analysis scanning electron microscope, is preferably equal to or more than 0.01 µm and equal to or less than 15 µm. The average value of the average length RSm is preferably equal to or more than 0.03 µm, more preferably equal to or more than 0.05 µm, further more preferably equal to or more than 0.08 µm, and even more preferably equal to or more than 0.10 µm. The average value of the average length RSm is preferably equal to or less than 10 µm, more preferably equal to or less than 5.0 um, and even more preferably equal to or less than 3.0 µm.

For the average value of the average length RSm of the roughness curve element, it is possible to optionally select three protrusions 8 at which the rough microstructure B is formed and to employ the average value of the average length RSm of the roughness curve elements of the three selected protrusions 8.

In the membrane carrier 3, the average length RSm of the roughness curve element of the flat portion 9 may be equal to or more than 0.01 um and equal to or less than 15 µm. The average length RSm of the roughness curve element of the flat portion 9 is preferably equal to or more than 0.03 µm, more preferably equal to or more than 0.05 µm, further more preferably equal to or more than 0.08 µm, and even more preferably equal to or more than 0.10 µm. The average length RSm of the roughness curve element of the flat portion 9 is preferably equal to or less than 10 µm, more preferably equal to or less than 5.0 µm, and further more preferably equal to or less than 3.0 µm.

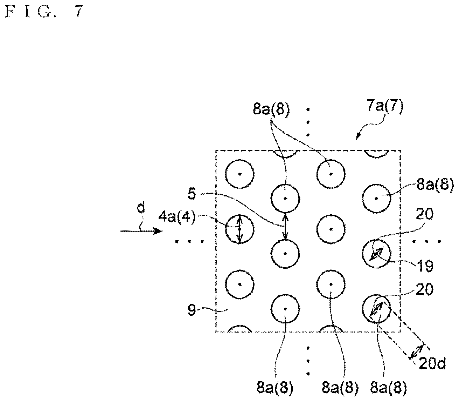

Fig. 7 is a diagram for explaining a method of measuring Rp and RSm of the protrusion 8 at which the rough microstructure B is formed in the rough structure A (7).

Using a three-dimensional roughness analysis scanning electron microscope, a roughness (concavity and convexity) profile is measured along the surface of the protrusion 8 (along a straight line 20 when viewed from the upper surface) having the center of the apex of the protrusion 8 (for example, a center 19 of the protrusion) as a central point. The straight line 20 is an optional straight line having a length of 20d, having the center of an apex (for example, the center 19 of the protrusion) as a central point. The length 20d is the same length as the diameter of the bottom surface of the protrusion 8. In a case where the straight line 20 is a straight line on the same plane (for example, in a case where both ends and the center are on the same plane), that is, in a case where the protrusion 8 is in a shape of a truncated cone, a truncated polypyramid, a cylinder, and a polygonal prism, the maximum peak height Rp of the roughness curve defined by JIS B0601: 2013 and the average length RSm of the roughness curve elements are calculated from the roughness profile. In a case where the straight line 20 is not a straight line on the same plane, that is, in a case where the protrusion 8 is in a shape of a cone, a polygonal pyramid, a hemisphere, and a hemi-ellipsoid, an inclination is corrected from the roughness profile and the maximum peak height Rp of the roughness curve defined by JIS B0601: 2013 as a plane and the average length RSm of the roughness curve elements are calculated.

The Rp and RSm in the protrusion 8 at which the rough microstructure B formed can be adjusted within the numerical value range by etching, photolithography, mechanical cutting, laser processing, and the like when the membrane carrier 3 having the rough structure A (7) is prepared by thermal imprinting. More specifically, it is preferable to adjust Rp and RSm in the protrusion 8 at which the rough microstructure B is formed by setting Rp and RSm on a surface of the mold used for thermal imprinting to predetermined values . For example, it is preferable to adjust Rp and RSm in the protrusion 8 at which the rough microstructure B is formed by etching, photolithography, mechanical cutting, polishing, laser processing, and the like on the surface of the mold. Examples of the polishing include cutting by dicing, sandblasting, and the like. In the laser processing, it is possible to adjust Rp and RSm by controlling an output of the laser.

That is, it is preferable that a method of manufacturing the test kit 18 according to the present embodiment includes a step (thermal imprinting step) of preparing the membrane carrier 3 having the rough structure A (7) by thermal imprinting. In the thermal imprinting step, the surface of the mold on which a plurality of concave portions are formed is applied to a base material made of a thermoplastic resin (for example, a membrane-shaped base material), for example, and the base material is heated to form a membrane carrier 3 having the rough structure A (7) (a plurality of protrusions 8) corresponding to the shape of the concave portion and the flat portion 9.

In an embodiment, at least one of a carbon atom and a nitrogen atom, and an oxygen atom may be present on the surface of the detection zone.

The ratio of the number of oxygen atoms to the total number of atoms of each atom (number of oxygen atoms/(number of carbon atoms + number of nitrogen atoms + number of oxygen atoms)) is equal to or more than 0.01 and equal to or less than 0.50. In the membrane carrier of one embodiment, the oxygen atomic number ratio (number of oxygen atoms/(number of carbon atoms + number of nitrogen atoms + number of oxygen atoms)) on the surface of the detection zone is equal to or more than 0.01, preferably equal to or more than 0.05, more preferably equal to or more than 0.10, and further more preferably equal to or more than 0.20. In the membrane carrier of one embodiment, the oxygen atomic number ratio (number of oxygen atoms/(number of carbon atoms + number of nitrogen atoms + number of oxygen atoms)) on the surface of the detection zone is equal to or less than 0.50, preferably equal to or less than 0.40, more preferably equal to or less than 0.38, further more preferably equal to or less than 0.36, and further more preferably equal to or less than 0.30, and even more preferably equal to or less than 0.10. The higher the oxygen atomic number ratio on the surface of the detection zone, the easier it is for the detection substance to adhere to the surface. As the detection substance is fixedly attached to the surface, the amount of the detection substance that is washed away when the liquid sample is developed is reduced, and highly sensitive test becomes possible. In a case where the oxygen atomic number ratio on the surface of the detection zone is equal to or less than the upper limit value, the occurrence of erroneous detection due to the reaction between the labeling substance and the detection substance when a solution not containing a substance to be detected is developed is further suppressed.

The oxygen atomic number ratio on the surface of the detection zone is calculated by X-ray photoelectron spectroscopy (XPS). The calculation of the oxygen atomic number ratio by XPS is described below. A binding energy correction of the spectrum obtained by the measurement is performed by a C-C bond in a C1s spectrum. A background (BG) is subtracted for each peak of the C1s spectrum, an N1s spectrum, and an O1s spectrum of the spectrum subjected to the binding energy correction. A peak area (signal intensity) of each atom calculated by subtracting the BG from each peak is divided by a correction coefficient (relative sensitivity coefficient, transmission function, and kinetic energy correction) and was calculated such that the total area after correction becomes 100. Each of the obtained values is defined as the number of carbon atoms, the number of nitrogen atoms, and the number of oxygen atoms, and the oxygen atomic number ratio (the number of oxygen atoms/ (the number of carbon atoms + the number of nitrogen atoms + the number of oxygen atoms)) is calculated.

The oxygen atomic number ratio on the surface of the detection zone can be adjusted within the range by surface-treating the surface of the detection zone. The surface treatment method is not limited at all, and various techniques such as various plasma treatments, corona treatments, UV irradiation, and surface modification by UV/ozone treatment may be used.

It is preferable that the surface treatment is performed only in the detection zone. By performing the method only in the detection zone, the detection substance is not fixedly attached to a non-detection zone (region other than the detection zone) in the flow channel, and the detection substance can be fixedly attached only to the detection zone with high efficiency. As a result, a detection signal can be easily recognized in the detection zone (the S/N ratio becomes high).

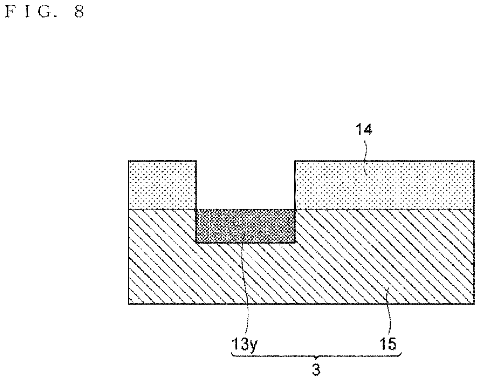

Examples of a method of selectively surface-treating the surface of the detection zone to modify the surface of the detection zone include a method of covering sites other than the detection zone with a shieldable mask (shield) and performing surface treatment on the exposed detection zone. Fig. 8 is a diagram for explaining a method of selectively surface-treating the surface of the detection zone. A shield 14 having a void portion is disposed on the membrane carrier 3 to expose the detection zone (surface treatment portion). Aportion of the membrane carrier 3 covered with the shield 14 becomes the untreated portion (non-detection zone) 15. As the shield 14, a metal plate is preferable. By surface-treating the exposed site, the membrane carrier 3 having an oxygen atomic number ratio on the surface of the detection zone within the range is obtainable.

In the embodiment, as the material of the membrane carrier, it is preferable to use a resin having a surface oxygen atomic number ratio (oxygen atomic number/(carbon atomic number + nitrogen atomic number + oxygen atomic number)) of less than 0.01, and it is more preferable to use a resin having a surface oxygen atomic number ratio of equal to or less than 0.005. The resin having an oxygen atomic number ratio on the surface of less than 0.01 is a resin that does not contain oxygen atoms in the structural formula of the main component, and may be a resin that contains carbon atoms such as a polyolefin-based resin, a polystyrene-based resin, a fluorine-based resin, and the like, and does not contain nitrogen atoms and oxygen atoms. Specific examples of such a resin include polyethylene (PE), cycloolefin polymer (COP), polypropylene (PP), polystyrene (PS), polyvinylidene fluoride (PVDF), and the like. The resin having an oxygen atomic number ratio on the surface of less than 0.01 may be a resin containing carbon atoms and nitrogen atoms and not containing oxygen atoms such as a polyimide resin. In a case where a resin containing carbon atoms and not containing nitrogen atoms and oxygen atoms is used, the oxygen atomic number ratio (number of oxygen atoms/(number of carbon atoms + number of nitrogen atoms + number of oxygen atoms)) in the detection zone is substantially equal to a value of the number of oxygen atoms/ (number of carbon atoms + number of oxygen atoms).

In a case where the oxygen atomic number ratio on the surface is equal to or less than 0.005, when a membrane carrier is prepared, a test kit is prepared using the prepared membrane carrier, and a liquid sample is developed, attachment of a labeling substance to a non-detection zone is further suppressed. In a case where the labeling substance is attached to the non-detection zone, even if a signal having the same intensity is generated in the detection zone, it becomes difficult to recognize thereof (the S / N ratio becomes low) .

In a case where the oxygen atomic number ratio on the surface is equal to or less than 0.005, when a membrane carrier is prepared, a test kit is prepared using the prepared membrane carrier, and a liquid sample is developed, attachment of a labeling substance to a non-detection zone is further suppressed. In a case where the labeling substance is attached to the non-detection zone, even if a signal having the same intensity is generated in the detection zone, it becomes difficult to recognize thereof (the S / N ratio becomes low) .

In the test kit 18 according to the present embodiment, the detection zone 3y of the membrane carrier 3 shows a change in color when the substance to be detected is detected. The change in color may be a change in color that can be confirmed by an optical technique.

Examples of the optical technique mainly include two techniques of visual determination and measurement of fluorescence or emission intensity. In a case of visual determination, a change in color preferably occurs such that color difference between two color stimuli at a time of measuring color before and after the detection by a color system of the CIE1976L*a*b* color space (ΔE described in JIS Z8781-4: 2013) is equal to or more than 0.5. In a case where the color difference is equal to or more than 0.5, it is easy to visually confirm the color difference. In a case where the fluorescence or emission intensity is measured and determined, the change in color preferably occurs such that a ratio (FI1/FI2) = equal to or more than 10/1, which is a ratio of fluorescence or emission intensity (FI1) in the detection zone 3y and fluorescence or emission intensity (FI2) in an upstream area and a downstream area adjacent to the detection zone 3y. In a case where the ratio is equal to or more than 10/1, separation of signal and noise becomes easy.

In order to prepare the detection zone 3y in the test kit 18 of the present embodiment, in an embodiment, a detection substance is immobilized on at least a part of the flow channel 2. That is, the detection substance for detecting a substance to be detected is immobilized in the detection zone 3y. The change in color in the detection zone 3y is caused by holding the substance to be detected in the detection zone 3y by the detection substance (reacting with the detection substance).

In other words, the method of manufacturing the test kit 18 includes a step of immobilizing the detection substance in the detection zone 3y, which causes a change in color by holding the substance to be detected in the detection zone 3y. From a viewpoint that the detection substance (reagent) can be more efficiently immobilized in the detection zone 3y, pre-surface treatment may be performed in a site provided with the detection zone 3y in the membrane carrier 3. As the surface treatment method, the method exemplified above can be used.

In the present embodiment, examples of the detection substance (reagent) include an antibody. The antibody is an antibody that reacts with a substance to be detected by an antigen-antibody reaction, and may be a polyclonal antibody or a monoclonal antibody.

The change in color in the detection zone 3y may be caused by a label having an antibody or an antigen-binding fragment thereof that specifically reacts with the substance to be detected in the liquid sample. The change in color occurs as the label is held in the detection zone 3y by the detection substance (reacts (binds) with the detection substance) and colored, for example.

The label may be a labeled antibody such as a fluorescently labeled antibody, a chemiluminescently labeled antibody, or an enzyme-labeled antibody, and may be one in which the antibody or the antigen-binding fragment thereof binds to a particle such as colloidal particles and latex particles. The antigen-binding fragment refers to a fragment that can specifically bind to a substance to be detected, for example, an antigen-binding fragment of an antibody. The label can bind to the substance to be detected via an antibody or an antigen-binding fragment thereof. The particles may have magnetism or fluorescent properties. Examples of the colloidal particles include metal colloidal particles such as gold colloidal particles and platinum colloidal particles. The particles are preferably latex particles in view of particle diameter control, dispersion stability, and ease of binding. Although the material of the latex particles is not limited, polystyrene is preferable.

The particles are preferably colored particles or fluorescent particles, and more preferably colored particles in view of visibility. The colored particles may be any particles as long as the color can be detected with naked eye. The fluorescent particles may contain a fluorescent substance. The particles may be colored latex particles or fluorescent latex particles. In a case where the particles are colored latex particles, the change in color described above is suitably determined visually. In addition, in a case where the particles are fluorescent latex particles, the change in color described above is suitably determined by measuring the fluorescence intensity.

Such label described above is provided in at least a part of the test kit 18 so as to react with the substance to be detected in the dropped liquid sample. The label may be provided in a member in the test kit 18 or may be provided in at least a part (upstream side from the detection zone 3y) of the flow channel 2 of the membrane carrier 3. The label that has reacted (bound) with the substance to be detected is held in the detection zone 3y by the detection substance (by the detection substance reacting (binding) with the substance to be detected). With this, a change in color (color development by the label) occurs in the detection zone 3y.

The test method of the liquid sample according to an aspect of the present embodiment is a test method using the test kit 18.

A test method of a liquid sample using the test kit 18 may include a step of mixing a liquid sample and a label specifically binding to a substance to be detected in the liquid sample, preparing a mixture solution sample (mixed liquid sample), and binding the substance to be detected and the label to each other; a step of dropping the mixture solution sample onto a dropping zone 3x provided in the membrane carrier 3; a step of transporting the mixture solution sample from the dropping zone 3x to the detection zone 3y by the rough structure A (7); and a step of detecting a change in color (color development by the label) in the detection zone 3y.

In addition, for example, the test method may include a step of dropping the liquid sample onto the dropping zone 3x on the surface of the membrane carrier 3; a step of transporting the liquid sample from the dropping zone 3x to the detection zone 3y through the rough structure A (7) by the capillary action by the rough structure A (7) (a plurality of protrusions 8) formed on the surface of the membrane carrier 3; and a step of detecting a change in color in the detection zone 3y (optically determining the presence or absence of a change in color) by binding the substance to be detected in the liquid sample to a label via the antibody or antigen-binding fragment thereof, and binding the substance to be detected to a reagent immobilized in the detection zone 3y, in the transporting process.

In a step of binding the substance to be detected and the label to each other of the test method, the method of mixing the liquid sample with the label is not limited. For example, the method may be a method of adding a liquid sample to a container containing the label, for example, or may be a method of mixing a liquid containing the label with the liquid sample. In addition, for example, a filter may be interposed between dropping ports of the container containing the liquid sample, and the label may be immobilized in the filter.

As described above, although the embodiments of the present invention have been described above with reference to the drawings, these are examples of the present invention, and various configurations other than the above may be employed.

### Examples

Hereinafter, the present embodiment will be specifically described with reference to examples and comparative examples, but the present embodiment is not limited to these examples.

### [Example 1]

### <Preparation of membrane carrier>

A cycloolefin polymer sheet (ZeonorZF-14, manufactured by Nippon Zeon Co., Ltd., film thickness 200 µm) was thermally imprinted, and a membrane carrier was prepared such that a conical-shaped protrusion 8 having a diameter of a bottom surface of a rough structure A (protrusion) (hereinafter, referred to as "diameter of the protrusion" or "diameter") of 30 µm and a height of the rough structure A (protrusion) (hereinafter, also referred to as "height") of 30 µm was arranged in a triangular array format as shown in Fig. 3 with an average distance between the protrusions of 50 µm.

Here, when performing thermal imprinting, the rough microstructure B was formed on the surface of the rough structure A (protrusion) by using an etching-treated mold, and the maximum peak height Rp of the roughness curve and the average length RSm of the roughness curve elements of the protrusions 8 at which the rough microstructure B is formed were adjusted to values shown in Table 1, respectively.

The types of molds used and the etching treatment conditions are as follows.

A nickel mold in which conical-shaped holes having a diameter of 30 um and a depth of 30 um at the entrance of the holes are arranged in a triangular array system with an average distance between the holes of 50 um was immersed in an etching solution (ferrous chloride solution) with a concentration of 40 mass% for 10 minutes, and the immersion time was adjusted to obtain a mold.

A three-dimensional roughness analysis electron microscope (ERA-600 manufactured by Elionix Inc.) was used to measure the maximum peak height Rp of the roughness curve and the average length RSm of the roughness curve elements (refer to Fig. 7).

Three conical-shaped protrusions 8 were optionally selected. The three protrusions 8 were observed at a magnification of 10,000 using a three-dimensional roughness analysis electron microscope to obtain a roughness-enhanced secondary electron image. Subsequently, a roughness profile of the straight line 20 having a length of 20d of 30 um was measured with the central portion of the apex of the protrusion 8 (central point 19 of the convex portion) as a central point. The roughness profiles of the three straight lines 20 were subjected to inclination correction (a long wavelength component (waviness component) is separated by a spline high-pass filter to obtain a cross-sectional curve), and the maximum peak height Rp of the roughness curve defined by JIS B0601: 2013 as a plane and the average length RSm of the roughness curve elements were calculated, respectively. The value obtained by averaging the three obtained data was used as the evaluation value.

### <Preparation of detection zone (detection portion)>

The surface of the obtained membrane carrier was irradiated with O₂ plasma under the conditions of output: 100 W, irradiation time: 120 seconds, and pressure: 6 × 10⁻² mbar. In this way, the surface-treated membrane carrier 3 was obtained.

### <Silane coupling agent treatment>

A silane coupling agent-treated solution having a methanol: distilled water: 3-aminopropyltriethoxysilane (also called APTES, manufactured by Tokyo Ohka Kogyo Co., Ltd.) = 92: 5: 3 (mass ratio) was prepared.

Subsequently, the surface-treated membrane carrier 3 was immersed in the silane coupling agent-treated solution at room temperature for 4 hours to attach the silane coupling agent to the surface of the membrane carrier 3. Subsequently, the membrane carrier 3 was rinsed with methanol and dried at 120°C for 5 minutes to obtain a membrane carrier 3 to which a silane coupling agent was attached.

### <Measurement of amount of antibody carried>

An anti-CRP antibody solution was prepared by diluting the fluorescently labeled anti-CRP antibody with PBS (phosphate buffered saline) and applied to the membrane carrier 3. After performing drying at 45°C for 1 hour, ultrasonic cleaning was performed in PBS containing 2 (v/v) % of Triton X-100 (product name) . The fluorescence intensity of the remaining antibody was evaluated using a fluorescence microscope (BZ-X710 manufactured by KEYENCE Corporation).

### [Examples 2 to 6]

The experiment was performed in the same manner as in Example 1 except that the maximum peak height Rp of the roughness curve of and the average length RSm of the roughness curve elements of the protrusion 8 at which the rough microstructure B is formed were adjusted to the values shown in Table 1, respectively, by adjusting the etching treatment conditions (specifically, the concentration of the etching solution or the immersion time) for the mold.

The obtained results are shown in Table 1.

### [Examples 7 to 12]

The experiment was performed in the same manner as in Example 1 except that the maximum peak height Rp of the roughness curve of and the average length RSm of the roughness curve elements of the protrusion 8 at which the rough microstructure B is formed were adjusted to the values shown in Table 1, respectively, by adjusting the etching treatment conditions for the mold, and 3-glycidoxypropyltrimethoxysilane (also referred to as GOPTS) was used instead of 3-aminopropyltriethoxysilane (also referred to as APTES) .

The obtained results are shown in Table 1.

### [Examples 13 to 18]

The experiment was performed in the same manner as in Example 1 except that the maximum peak height Rp of the roughness curve of and the average length RSm of the roughness curve elements of the protrusion 8 at which the rough microstructure B is formed were adjusted to the values shown in Table 2, respectively, by adjusting the etching treatment conditions for the mold, and the following cross-linking agent treatment was carried out.

The obtained results are shown in Table 2.

### (Cross-linking agent treatment)

A glutaraldehyde solution was prepared by adding 2.5% of glutaraldehyde to PBS.

Subsequently, the membrane carrier 3 treated with the silane coupling agent was immersed in a glutaraldehyde solution at room temperature for 1 hour, and glutaraldehyde (also referred to as GA) was attached to the surface of the membrane carrier 3. Subsequently, the membrane carrier 3 was rinsed with ultrapure water and then dried at 45°C for 30 minutes to obtain a membrane carrier 3 to which glutaraldehyde was attached.

### [Comparative Example 1]

The experiment was performed in the same manner as in Example 1 except that the silane coupling agent treatment was not performed.

The obtained results are shown in Table 2.

### [Comparative Example 2]

The experiment was performed in the same manner as in Example 1 except that the maximum peak height Rp of the roughness curve of and the average length RSm of the roughness curve elements of the protrusion 8 at which the rough microstructure B is formed were adjusted to the values shown in Table 2, respectively, by adjusting the etching treatment conditions for the mold.

The obtained results are shown in Table 2.

### [Comparative Example 3]

The experiment was performed in the same manner as in Example 1 except that a mold not subjected to etching treatment was used and the silane coupling agent treatment was not performed. The obtained results are shown in Table 2.

### [Comparative Example 4]

The experiment was performed in the same manner as in Example 1 except that a mold not subjected to etching treatment was used. The obtained results are shown in Table 2.

### [Comparative Example 5]

The experiment was performed in the same manner as in Example 13 except that the silane coupling agent treatment was not performed.

The obtained results are shown in Table 2.

**Table 1**

| | Example 1 | Example 2 | Example 3 | Example 4 | Example 5 | Example 6 | Example 7 | Example 8 | Example 9 | Example 10 | Example 11 | Example 12 |
|---|---|---|---|---|---|---|---|---|---|---|---|---|
| Rp of Protrusion [µm] | 0.25 | 0.026 | 0.092 | 0.19 | 6.5 | 0.0098 | 0.25 | 0.026 | 0.092 | 0.19 | 6.5 | 0.0098 |
| RSm of Protrusion [µm] | 2.3 | 1.1 | 0.24 | 0.12 | 0.062 | 12 | 2.3 | 1.1 | 0.24 | 0.12 | 0.062 | 12 |
| Silane Coupling Agent | APTES | APTES | APTES | APTES | APTES | APTES | GOPTS | GOPTS | GOPTS | GOPTS | GOPTS | GOPTS |
| Cross-linking Agent | None | None | None | None | None | None | None | None | None | None | None | None |
| Fluorescence Intensity [a.u.] | 220 | 38 | 134 | 225 | 59 | 30 | 111 | 29 | 36 | 72 | 42 | 23 |

**Table 2**

| | Example 13 | Example 14 | Example 15 | Example 16 | Example 17 | Example 18 | Comparative Example 1 | Comparative Example 2 | Comparative Example 3 | Comparative Example 4 | Comparative Example 5 | |
|---|---|---|---|---|---|---|---|---|---|---|---|---|
| Rp of Protrusion [µm] | 0.25 | 0.026 | 0.092 | 0.19 | 6.5 | 0.0098 | 0.25 | 0.002 | - | - | 0.25 | |
| RSm of Protrusion [µm] | 2.3 | 1.1 | 0.24 | 0.12 | 0.062 | 12 | 2.3 | 18 | - | - | 2.3 | |
| Silane Coupling Agent | APTES | APTES | APTES | APTES | APTES | APTES | None | APTES | None | APTES | None | |
| Cross-linking Agent | GA | GA | GA | GA | GA | GA | None | None | None | None | GA | |
| Fluorescence Intensity [a.u.] | 242 | 65 | 171 | 240 | 68 | 45 | 6 | 10 | 5 | 10 | 9 | |

From the results of Tables 1 and 2, it was found that in the membrane carrier for a test kit according to the present embodiment, as the Rp and RSm of the protrusion of the rough structure A are within specific ranges, respectively, and a rough microstructure B to which a silane coupling agent is attached is formed, an amount of an antibody carried which is a detection substance increases, and a substance to be detected can be detected with high sensitivity.

### REFERENCE SIGNS LIST

2 flow channel
3 membrane carrier
3x dropping zone
3y detection zone (detection portion)
4, 4a, 4b, 4c, 4d representative length on the bottom surface of the protrusion (diameter of the bottom surface of the protrusion)
5 distance between adjacent protrusions
6, 6a, 6b, 6c, 6d height of protrusion
7, 7a, 7b, 7c, 7d rough structure A
8, 8a, 8b, 8c, 8d protrusion
9 flat portion
10, 10a, 10b, 10c, 10d bottom surface of protrusion
13y detection zone
14 shield
15 untreated portion
18 test kit
18a housing
18b first opening
18c second opening
19 center of protrusion
20 straight line passing through the center of the protrusion
20d length of a straight line passing through the center of the protrusion
d flow direction of liquid sample (transporting direction)

## Claims

1. A membrane carrier for a test kit for detecting a substance to be detected in a liquid sample, the membrane carrier comprising:
a flow channel having a detection zone,
wherein the flow channel has a rough structure A having protrusions,
at least in the detection zone, a rough microstructure B is formed on a surface of the protrusion,
a silane coupling agent is attached to a surface of the rough microstructure B, and
a maximum peak height Rp of a roughness curve of the protrusion at which the rough microstructure B is formed, as measured according to JIS B0601: 2013 using a three-dimensional roughness analysis scanning electron microscope, is equal to or more than 0.005 µm and equal to or less than 10 µm, and an average length RSm of a roughness curve element is equal to or more than 0.01 µm and equal to or less than 15 µm.

2. The membrane carrier for a test kit according to Claim 1,
wherein the silane coupling agent includes at least one selected from the group consisting of a silane coupling agent having an amino group, a silane coupling agent having a mercapto group, a silane coupling agent having an epoxy group, a silane coupling agent having an acrylic group, a silane coupling agent having a methacryl group, a silane coupling agent having a vinyl group, and a silane coupling agent having an isocyanate group.

3. The membrane carrier for a test kit according to Claim 1 or 2,
wherein a cross-linking agent is further attached to the surface of the rough microstructure B.

4. The membrane carrier for a test kit according to Claim 3,
wherein the cross-linking agent includes at least one selected from glutaraldehyde, dextran, 1,4-phenyldiisocyanate, toluene-2,4 diisocyanate, polyethyleneimine, 1-ethyl-3-(3-dimethylaminopropyl)carbodiimide, N-succinimidyl 4-(N-maleimide methyl)cyclohexane-1-carboxylate, and N-hydroxysucciimide.

5. The membrane carrier for a test kit according to any one of Claims 1 to 4,
wherein the rough structure A includes a thermoplastic resin.

6. The membrane carrier for a test kit according to Claim 5,
wherein the thermoplastic resin includes at least one selected from a polyester-based resin, a polyolefin-based resin, a polystyrene-based resin, a polycarbonate-based resin, a fluorine-based resin, and a (meth)acrylic-based resin.

7. The membrane carrier for a test kit according to any one of Claims 1 to 6,
wherein an average height of the protrusions is equal to or more than 5 µm and equal to or less than 1,000 µm.

8. The membrane carrier for a test kit according to any one of Claims 1 to 7,
wherein an average distance between the adjacent protrusions in the rough structure A is equal to or less than 500 µm.

9. The membrane carrier for a test kit according to any one of Claims 1 to 8,
wherein an average diameter of a bottom surface of the protrusion is equal to or more than 5 µm and equal to or less than 1,000 µm.

10. A test kit having the membrane carrier according to any one of Claims 1 to 9.

## Patentansprüche

1. Membranträger für einen Testkit zum Nachweisen einer Substanz, die in einer flüssigen Probe nachgewiesen werden soll, wobei der Membranträger umfasst:
einen Fließkanal mit einer Nachweiszone,
wobei der Fließkanal eine raue Struktur A mit Vorsprüngen aufweist,
mindestens in der Nachweiszone eine raue Mikrostruktur B auf einer Fläche des Vorsprungs ausgebildet ist,
ein Silan-Kupplungsmittel an einer Fläche der rauen Mikrostruktur B angebracht ist und eine maximale Peakhöhe Rp einer Rauheitskurve des Vorsprungs, an dem die raue Mikrostruktur B ausgebildet ist, wie gemäß JIS B0601: 2013 unter Verwendung eines dreidimensionalen Rauheitsanalysescan-Elektronenmikroskops gemessen, gleich zu oder größer als 0,005 µm und gleich zu oder kleiner als 10 µm ist, und eine durchschnittliche Länge RSm eines Rauheitskurvenelements gleich zu oder größer als 0,01 µm und gleich zu oder kleiner als 15 µm ist.

2. Membranträger für einen Testkit gemäß Anspruch 1,
wobei das Silan-Kupplungsmittel mindestens eines, ausgewählt aus der Gruppe, bestehend aus einem Silan-Kupplungsmittel mit einer Aminogruppe, einem Silan-Kupplungsmittel mit einer Mercaptogruppe, einem Silan-Kupplungsmittel mit einer Epoxygruppe, einem Silan-Kupplungsmittel mit einer Acrylgruppe, einem Silan-Kupplungsmittel mit einer Methacrylgruppe, einem Silan-Kupplungsmittel mit einer Vinylgruppe und einem Silan-Kupplungsmittel mit einer Isocyanatgruppe, beinhaltet.

3. Membranträger für einen Testkit gemäß Anspruch 1 oder 2,
wobei ein Vernetzungsmittel ferner an die Fläche der rauen Mikrostruktur B angebracht ist.

4. Membranträger für einen Testkit gemäß Anspruch 3,
wobei das Vernetzungsmittel mindestens eines, ausgewählt aus Glutaraldehyd, Dextran, 1,4-Phenyldiisocyanat, Toluol-2,4-diisocyanat, Polyethylenimin, 1-Ethyl-3-(3-dimethylaminopropyl)carbodiimid, N-Succinimidyl-4-(N-maleimidmethyl)cyclohexan-1-carboxylat und N-Hydroxysuccinimid, beinhaltet.

5. Membranträger für einen Testkit gemäß einem jeglichen der Ansprüche 1 bis 4,
wobei die raue Struktur A ein thermoplastisches Harz beinhaltet.

6. Membranträger für einen Testkit gemäß Anspruch 5,
wobei das thermoplastische Harz mindestens eines, ausgewählt aus einem Harz auf Polyesterbasis, einem Harz auf Polyolefinbasis, einem Harz auf Polystyrolbasis, einem Harz auf Polycarbonatbasis, einem Harz auf Fluorbasis und einem Harz auf (Meth)acrylbasis, beinhaltet.

7. Membranträger für einen Testkit gemäß einem jeglichen der Ansprüche 1 bis 6, wobei eine durchschnittliche Höhe der Vorsprünge gleich zu oder größer als 5 µm und gleich zu oder kleiner als 1000 µm ist.

8. Membranträger für einen Testkit gemäß einem jeglichen der Ansprüche 1 bis 7, wobei ein durchschnittlicher Abstand zwischen den benachbarten Vorsprüngen in der rauen Struktur A gleich zu oder kleiner als 500 µm ist.

9. Membranträger für einen Testkit gemäß einem jeglichen der Ansprüche 1 bis 8, wobei ein durchschnittlicher Durchmesser einer Bodenfläche des Vorsprungs gleich zu oder größer als 5 µm und gleich zu oder kleiner als 1000 µm ist.

10. Testkit, der den Membranträger gemäß einem jeglichen der Ansprüche 1 bis 9 aufweist.

## Revendications

1. Support de membrane pour un kit d'essai destiné à détecter une substance à détecter dans un échantillon liquide, le support de membrane comprenant :
un canal d'écoulement comportant une zone de détection,
le canal d'écoulement comportant une structure rugueuse A comportant des saillies,
au moins dans la zone de détection, une microstructure B rugueuse est formée sur une surface de la saillie,
un agent de couplage de silane est fixé à une surface de la microstructure rugueuse B, et
une hauteur maximale de crête Rp d'une courbe de rugosité de la saillie à laquelle la microstructure rugueuse B est formée, mesurée selon JIS B0601 : 2013 à l'aide d'un microscope électronique à balayage à analyse de rugosité tridimensionnelle, est égale ou supérieure à 0,005 µm et égale ou inférieure à 10 µm, et une longueur moyenne RSm d'un élément de courbe de rugosité est égale ou supérieure à 0,01 µm et égale ou inférieure à 15 µm.

2. Support de membrane pour un kit d'essai selon la revendication 1,
dans lequel l'agent de couplage de silane comprend au moins un choisi parmi le groupe constitué d'un agent de couplage de silane comportant un groupe aminé, un agent de couplage de silane comportant un groupe mercapto, un agent de couplage de silane comportant un groupe époxy, un agent de couplage de silane comportant un groupe acrylique, un agent de couplage de silane comportant un groupe méthacryle, un agent de couplage de silane comportant un groupe vinyle et un agent de couplage de silane comportant un groupe isocyanate.

3. Support de membrane pour un kit d'essai selon la revendication 1 ou 2,
dans lequel un agent de réticulation est en outre fixé à la surface de la microstructure rugueuse B.

4. Support de membrane pour un kit d'essai selon la revendication 3,
dans lequel l'agent de réticulation comprend au moins un produit choisi parmi glutaraldéhyde, dextrane, 1,4-phényldiisocyanate, toluène-2,4 diisocyanate, polyéthylèneimine, 1-éthyl-3-(3-diméthylaminopropyl)carbodiimide, N-succinimidyl 4-(N-maléimide méthyl)cyclohexane-l-carboxylate, et N-hydroxysucciimide.

5. Support de membrane pour un kit d'essai selon l'une quelconque des revendications 1 à 4,
dans lequel la structure rugueuse A comprend une résine thermoplastique.

6. Support de membrane pour un kit d'essai selon la revendication 5,
dans lequel la résine thermoplastique comprend au moins un choisi parmi une résine à base de polyester, une résine à base de polyoléfine, une résine à base de polystyrène, une résine à base de polycarbonate, une résine à base de fluor et une résine à base de (méth)acrylique.

7. Support de membrane pour un kit d'essai selon l'une quelconque des revendications 1 à 6,
dans lequel une hauteur moyenne des saillies est égale ou supérieure à 5 µm et égale ou inférieure à 1000 µm.

8. Support de membrane pour un kit d'essai selon l'une quelconque des revendications 1 à 7,
dans lequel une distance moyenne entre les saillies adjacentes dans la structure rugueuse A est égale ou inférieure à 500 µm.

9. Support de membrane pour un kit d'essai selon l'une quelconque des revendications 1 à 8,
dans lequel un diamètre moyen d'une surface inférieure de la saillie est égal ou supérieur à 5 µm et égal ou inférieur à 1000 µm.

10. Kit d'essai comportant le support de membrane selon l'une quelconque des revendications 1 à 9.
